# FASCICULE DE BREVET EUROPEEN

(11) **EP 0 870 498 B1**
(45) Date de publication et mention de la délivrance du brevet: **11.05.2005**
(21) Numéro de dépôt: 98400647.8
(22) Date de dépôt: 19.03.1998
(51) Int. Cl.: A61K 9/70, A61K 7/00

(54) **Patch pharmaceutique, cosmétique ou dermo-pharmaceutique pour la délivrance de plusieurs composés actifs de nature différente**
Pharmazeutischer, kosmetischer oder dermo-pharmazeutischer Patch zur Verabreichung mehrerer verschiedenartiger Aktivstoffe
Pharmaceutical, cosmetic or dermo-pharmaceutical path for the delivery of a multitude of different active compounds

(30) Priorité: 11.04.1997 FR 9704498
(43) Date de publication de la demande: 14.10.1998
(73) Titulaire: L'OREAL, 75008 Paris (FR)
(72) Inventeur: Gueret, Jean-Louis H., 75018 Paris (FR)
(74) Mandataire: Boulard, Denis

(56) Documents cités:
- EP-A- 0 314 528
- EP-A- 0 764 441
- DE-A- 4 210 165
- FR-A- 2 735 024

## Description

La présente invention concerne un patch permettant la libération contrôlée dans l'épiderme d'au moins deux composés pharmaceutiquement, cosmétiquement ou dermo-pharmaceutiquement actifs, notamment de composés présentant des solubilités différentes. Selon l'invention il s'agit, en particulier, d'un premier groupe d'actifs liposolubles, et d'un second groupe d'actifs hydrosolubles.

Dans description de la présente invention ci-après, on désigne par le terme *"patch"* un système de délivrance d'actif présentant une structure composite sous forme de couches qui, par application sur la peau, assure la libération du produit actif par transdermie.

En ce qui concerne le composé actif liposoluble, il s'agit notamment d'un composé, apte à être solubilisé dans un milieu apolaire tel qu'un milieu huileux.

Quant au compose actif hydrosoluble, il se présente sous forme solide, notamment sous forme de poudre particulaire ou sous forme de granulés.

Il est connu d'utiliser des patchs permettant, par transdermie, de faire pénétrer des composés actifs.

De tels patchs présentent en général une structure comportant plusieurs couches successives dans l'ordre suivant: une première couche, dite couche support, généralement occlusive, c'est-à-dire constituée d'une matière imperméable au composé actif de façon à empêcher l'évaporation de ce dernier et faciliter la transdermie ; une seconde couche, dite couche de polymère, fixée sur la couche support et contenant le composé actif, cette couche pouvant venir directement au contact de la peau ; éventuellement, pour faciliter la fixation du patch sur la peau, une couche d'une matière adhésive appliquée à la surface de la couche de polymère et perméable au composé actif ; enfin, une couche détachable de protection, recouvrant de façon hermétique la couche de polymère, de façon à la protéger de toute contamination extérieure pendant le temps de stockage préalable à l'utilisation du patch.

On connaît, en particulier selon le document US-A-5 232 702, une structure de patch constituée d'une couche support occlusive et comprenant, fixée sur cette dernière, une couche de polymère en une matrice d'un polymère de silicone incluant, à l'état dispersé, des corps gras et des composés actifs hydrophiles. Si cette forme de patch est plus particulièrement appropriée pour la délivrance de composés actifs hydrosolubles, ce document ne décrit pas la délivrance de composés actifs supplémentaires de nature lipophile.

Par ailleurs, il a été décrit dans le document WO96/14 822 un patch occlusif de forme adaptée pour la traitement des rides du genre précité, comportant dans une matrice anhydre un composé actif hydrosoluble sous forme de poudre, cette matrice pouvant contenir, en outre, une huile, utilisée en tant qu'agent de pénétration.

De plus, le document FR-A-2 738 744 décrit un patch pour la libération contrôlée d'au moins un composé cosmétiquement ou dermo-pharmaceutiquement actif, comportant une couche réservoir, fixée sur une couche support, la couche réservoir étant constituée d'une matrice polymérique hydrophobe dans laquelle sont dispersées des particules du composé actif éventuellement instable en milieu oxydant et des particules d'au moins un agent hydro-absorbant, la couche réservoir étant anhydre.

On connaît, en outre, par le document DE-A-42 10 165, un patch constituée d'une matrice de silicone contenant un actif liposoluble, un actif hydrosoluble, et une huile augmentant la pénétration de l'actif liposoluble, cet actif hydrosoluble étant sous forme particulaire, dispersé dans la matrice.

Là présente invention propose un système de délivrance contrôlée comportant une matrice polymérique anhydre dans laquelle est dispersée une huile sous forme de gouttelettes, cette matrice étant destinée à libérer simultanément des composés actifs lipophiles et des composés actifs hydrophiles. L'utilisation d'une matrice polymérique anhydre dans laquelle est dispersée une huile, présente l'avantage de pouvoir obtenir un patch particulièrement souple, destiné à adhérer aux surfaces cutanées non planes, à fort rayon de courbure, et de ne pas être gêné par des zones de pliure de la peau.

Des essais effectués par la demanderesse ont montré que, dans certain cas des patchs antérieurs, l'adhésion sur la peau était difficile à assurer, ce qui est le cas, notamment lorsqu'on incorpore un proportion relativement importante d'huile.

De façon tout à fait surprenante, il a été trouvé que l'adjonction dans la matrice d'un agent hydroabsorbant conduisait à une amélioration sensible du pouvoir d'adhésion du patch de l'invention.

Par ailleurs, la demanderesse a constaté, qu'en dispersant, dans une matrice polymérique hydrophobe spécifique, une quantité appropriée d'huile, dans laquelle était dissous un premier composé actif lipophile, il était possible d'obtenir un patch comportant une couche de polymère anhydre, mais permettant néanmoins une excellente libération contrôlée d'un second composé actif pulvérulent de nature hydrophile.

Le patch permet, notamment, le conditionnement et l'administration contrôlée d'un ensemble de substances nutritives et/ou réparatrices de la peau de nature lipophile et hydrophile, et notamment d'actifs lipophiles et hydrophiles ayant tendance de s'inactiver mutuellement, lorsqu'il se trouvent en association.

Il a été constaté, en outre, d'une manière tout à fait surprenante et inattendue, qu'il était possible de conditionner dans une même matrice polymérique un (ou plusieurs) composé(s) actif(s) hydrophile(s), un (ou plusieurs) composé(s) actif(s) lipophile(s), et une (ou plusieurs) huile(s). Ainsi, il était possible de réaliser, par exemple, une association stable de particules d'un composé hydrosoluble, instable en milieu aqueux ou à l'air libre et/ou d'un composé actif liposoluble instable également en milieu aqueux ou à l'air libre, à une huile insaturée, siccative à l'air libre, sans risque de dégradation des composés actifs et sans risque de durcissement de l'huile.

Ainsi, la présente invention a pour objet un patch pharmaceutique, cosmétique ou dermo-pharmaceutique, comportant une couche de polymère hydrophobe, fixée sur une couche support et contenant : a) des premières particules d'au moins un composé actif hydrosoluble, b) des deuxièmes particules d'huile, c) au moins un composé actif liposoluble, caractérisé en ce que la couche de polymère contient, en outre, des troisièmes particules d'un agent hydro-absorbant, dispersées de façon homogène dans la couche de polymère.

Selon un premier mode de réalisation, le composé actif liposoluble est solubilisé dans l'huile.

Selon un autre mode de réalisation, le composé actif liposoluble se présente sous forme particulaire, sous forme de poudre ou de granulés, dispersé dans la couche de polymère.

L'huile utilisable conformément à l'invention, peut être choisi parmi les huiles suivantes, seules ou en mélange, parmi lesquelles on peut citer : les huiles d'origine animale, végétale ou minérale, et notamment les huiles animales ou végétales formées par des esters d'acide gras et de polyols, en particulier les triglycérides liquides, par exemple les huiles de tournesol, de maïs, de soja, de courge, de pépins de raisin, de sésame, de noisette, de pistache, d'abricot, d'amandes ou d'avocat; les huiles de poisson, le tricaprocaprylate de glycérol, ou les huiles végétales ou animales de formule R₁COOR₂ dans laquelle R₁ représente le reste d'un acide gras supérieur comportant de 7 à 19 atomes de carbone et R₂ représente une chaîne hydrocarbonée ramifiée contenant de 3 à 20 atomes de carbone, par exemple, l'huile de Purcellin ; l'huile de germes de blé, de calophyllum, de sésame, de coriandre, de carthame, l'huile de passiflore, l'huile de rosa mosqueta, de macadamia, de pépins de fruits (raisin, cassis, orange, kiwi), de colza, de coprah, d'arachide, d'onagre, de palme, de ricin, de lin, de jojoba, de chia, d'olive ou de germes de céréales comme l'huile de germes de blé, l'huile de son de riz, l'huile de karité ; des acétylglycérides; des octanoates, décanoates ou ricinoléates d'alcools ou de polyalcools; des triglycérides d'acides gras; des glycérides ; l'huile de paraffine, de vaseline, le perhydrosqualène ; des alcools gras (alcool stéarylique, alcool cétylique) et des acides gras (acide stéarique) et leurs esters ; les polyalkyl(C₁-C₂₀) siloxanes et notamment ceux à groupements terminaux triméthylsilyle, de préférence ceux dont la viscosité est inférieure à 0,06 m²/s parmi lesquels on peut citer les polydiméthylsiloxanes linéaires et les alkylméthylpolysiloxanes tels que la cétyldiméthicone (nom CTFA).

On peut encore citer les huiles hydrocarbonées partiellement fluorées ou les huiles perfluorées, et notamment les perfluoropolyéthers et les perfluoroalcanes.

La phase huileuse, c'est-à-dire les gouttelettes d'huile dispersées dans la couche de polymère, est présente dans une proportion allant de 0,1% à 30% en poids par rapport au poids total de la composition. De façon préférentielle, ce pourcentage est compris entre 5 et 25.

Lorsque les compositions selon l'invention sont utilisées pour le traitement cosmétique de la peau ou dans un but dermatologique, le composé actif contenu dans la phase huileuse est, plus particulièrement, choisi parmi les agents antioxydants, anti-radicaux libres, hydratants, dépigmentants, liporégulateurs, amincissants, anti-acnéiques, anti-psoriatiques, antiséborrhéiques, antihistaminiques, anti-vieillissement, anti-rides, kératolytiques, anti-inflammatoires, rafraichissants, cicatrisants, protecteurs vasculaires, antibactériens, antifongiques, antiperspirants, conditionneurs de la peau, immunomodulateurs et nourrissants.

Parmi les actifs lipophiles dissous dans la phase huileuse, utilisables pour un traitement par transdermie dans le cadre de la présente invention, on peut citer comme exemples, notamment les composés suivants :

D-α-tocophérol, DL-α-tocophérol, acétate de D-α-tocophérol, acétate de DL-α-tocophérol, palmitate d'ascorbyle, vitamine F et glycérides de vitamine F, vitamine D, vitamine D₂, vitamine D₃, rétinol, esters de rétinol, palmitate de rétinol, propionate de rétinol, β-carotène, D-panthénol, farnesol, acétate de farnesyle ; huiles de jojoba et de cassis riches acides gras essentiels ; acide n-octanoyl-5 salicylique et ses esters, acide salicylique et ses esters ; alkylesters d'a-hydroxyacides tels que l'acide citrique, acide lactique, acide glycolique ; acide asiatique, acide madécassique, asiaticoside, extrait total de centella asiatica, acide β-glycyrrhétinique, α-bisabolol, céramides comme le 2 oleoylamino-1,3 octadécane ; phytanetriol, phospholipides d'origine marine riches en acides gras essentiels polyinsaturés, éthoxyquine ; extrait de romarin, extrait de mélisse, quercetine, extrait de microalgues séchées, les anti-inflammatoires, notamment stéroïdiens, ainsi que les agents biostimulants comme certaines hormones ou des composée des synthèse de lipides et/ou de protéines.

La concentration du composé actif lipophile dans la phase huileuse que l'on peut utiliser dépend de la nature de l'huile végétale utilisée. Typiquement, cette concentration peut aller de 0,01 % à 20 % en poids par rapport au poids total de la phase huileuse.

Le cas échéant, la phase huileuse peut comporter, en outre, des agents tensioactifs lipophiles, par exemple le distéarate de sucrose, le distéarate de diglycéryle, le tristéarate de tétraglycéryle, le décastéarate de décaglycéryle, le monostéarate de diglycéryle, le tristéarate d'hexaglycéryle, le pentastéarate de décaglycéryle, le monostéarate de sorbitane, le tristéarate de sorbitane, le monostéarate de diéthylène glycol, l'ester de glycérol et d'acides palmitique et stéarique, le monostéarate polyoxyéthylèné 2 OE (comportant 2 motifs oxyéthylène), le mono et dibéhénate de glycéryle, le tétrastéarate de pentaérythritol.

Les particules d'un (ou plusieurs) composé(s) actif(s) solide(s) , hydrosoluble(s), lorsque les compositions conformes à l'invention sont utilisées pour le traitement cosmétique de la peau ou dans un but dermatologique, sont choisis parmi les actifs hydrophiles classiquement utilisés comme les agents antioxydants, anti-radicaux libres, hydratants, dépigmentants, liporégulateurs, amincissants, anti-acnéiques, antiseborrheiques, anti-vieillissement, anti-rides, kératolytiques, anti-inflammatoires, rafraichissants, cicatrisants, protecteurs vasculaires, antibactériens, antifungiques, antiperspirants, déodorants, conditionneurs de la peau, immunomodulateurs et nourrissants.

Dans les patchs selon la présente invention, le composé actif hydrophile, peut être choisi, par exemple, parmi l'acide ascorbique et ses sels biologiquement compatibles, les enzymes, les antibiotiques tels que le phosphate de clindamycine, les composants à effet tenseur tels que les poudres de protéines de soja ou de blé, les α-hydroxy acides et leurs sels, les polyacides hydroxylés, les sucroses et leurs dérivés, l'urée, les aminoacides, les oligopeptides, les extraits végétaux hydrosolubles, et de levures, les hydrolysats de protéines telles que la collagène et l'élastine, l'acide hyaluronique, les mucopolysaccharides, les vitamines B₂, B₆, H, PP, le panthénol, l'acide folique, l'acide acétyl salicylique l'allantoïne, l'acide glycyrrhétique, l'acide kojique, l'hydroquinone, etc..

Avantageusement, le composé actif hydrosoluble est présent dans la couche de polymère dans une proportion allant de 0,2% à 20% en poids, par rapport au poids total de ladite couche.

De manière préférentielle, les particules du composé actif hydrosoluble présentent une taille moyenne comprise entre 0,2 µm et 0,5 mm.

Selon un aspect intéressant de l'invention, il est possible de conditionner dans une même matrice un (ou plusieurs) composé(s) actif(s) hydrophile(s), sensible(s) à l'hydrolyse, un (ou plusieurs) composé(s) actif(s) lipophile(s), sensible(s) à l'oxydation, et une (ou plusieurs) huile(s), sensible(s) à l'oxydation également. Dans ces conditions, il est possible, en outre, d'utiliser un actif hydrosoluble, incompatible avec le composé actif liposoluble ou avec l'huile, dans des préparations topiques classiques du type crème ou lotion. Ainsi, il est possible, par exemple, de réaliser une association de particules de vitamine C à une huile insaturée contenant la vitamine A sans risque de dégradation des vitamines et sans risque de polymérisation de l'huile.

Avantageusement, la matrice formant la couche de polymère peut contenir des particules d'au moins un agent hydro-absorbant dispersées de façon homogène dans ladite matrice. En effet, au contact de l'humidité de la peau, les particules de l'agent hydro-absorbant captent de l'eau, favorisant ainsi la solubilisation du composé actif solide, hydrosoluble. En quelque sorte, par cette solubilisation "in situ" de l'actif hydrosoluble, sa biodisponibilité est quasi instantanée, et toute interaction éventuelle avec les autres composés présents dans la couche polymérique est minimisée. L'humidité de la peau peut jouer le rôle de solubilisant de l'actif hydrosoluble d'autant plus, que la couche support du patch crée des conditions occlusives.

Parmi les agents hydro-absorbants pouvant être présents dans la matrice polymérique hydrophobe à l'état dispersé, on peut citer, de préférence, les polyacrylates réticulés superabsorbants à fort taux de gonflement dans l'eau, tels que ceux commercialisés par la Société NORSOLOR sous la dénomination *Aquakeep*® ; l'alcool polyvinylique ; les polymères carboxyvinyliques tels que ceux commercialisés par la Société GOODRICH sous les dénominations de "Carbopol"® ; les dérivés semi-synthétiques de la cellulose tels que la carboxyméthylcellulose ; les substances naturelles telles que les amidons, les gommes naturelles (gomme de guar, gomme arabique, gomme adragante), la caséine, les phytocolloïdes (carragénates, alginates, agar-agar), les fibres de coton et la gélatine.

Il est possible également, d'utiliser un agent hydro-absorbant sous forme de particules d'une poudre d'émulsion lyophilisée ou atomisée. Avantageusement, les particules de l'agent hydro-absorbant ont une taille moyenne comprise entre environ 0,2 µm et environ 1,5 mm.

On préfère tout particulièrement utiliser selon l'invention les polyacrylates réticulés "superabsorbants" dont la présence à l'état dispersé dans la matrice polymérique hydrophobe favorise, après hydratation, une meilleure diffusion du composé actif hydrophile. Grâce à la présence de ces agents "superabsorbants", on obtient, en outre un meilleur collage du patch sur la peau, notamment lorsqu'une application prolongée est souhaitée.

L'agent hydro-absorbant tel que défini ci-dessus est présent, de préférence, dans une proportion allant d'environ 0,1 % à environ 30 % en poids, et plus particulièrement allant de 0,5 % à 10 % par rapport au poids total de la couche de polymère.

Dans les patchs selon la présente invention, la matrice polymérique hydrophobe est par exemple à base d'un polymère de silicone ou d'un polyuréthanne du type polyester polyuréthanne ou polyéther polyuréthanne.

Lorsque la matrice polymérique est à base d'un polymère de silicone, le prépolymère de silicone est de préférence choisi parmi les organopolysiloxanes linéaires substitués sur l'atome de silicium par des groupes choisis parmi un groupe alkyle en C₁-C₆, aryle ou ar(alkyle en C₁-C₂), les atomes de silicium terminaux étant trisubstitués. De tels organopolysiloxanes sont décrits notamment dans les brevets US-2 541 137, 2 723 966, 2 863 846, 2 890 188, 2 927 907, 3 002 951 et 3 035 016.

On préfère en particulier un prépolymère de silicone comportant des motifs suivants : 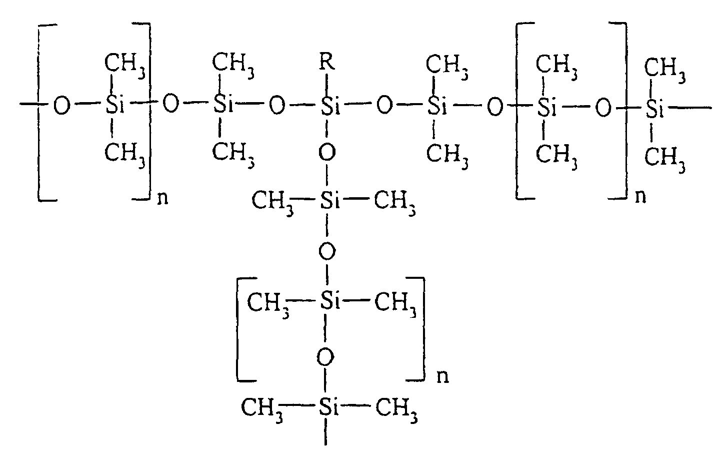 dans lesquels :
R représente un groupe alkyle ou alkoxy contenant de 1 à 7 atomes de carbone, un groupe vinyle ou phényle, et dans lesquels n est choisi dans la gamme allant d'environ 100 à environ 5 000.

Parmi les organopolysiloxanes particulièrement préférés selon l'invention, on peut citer ceux connus sous les dénominations de SILASTIC 382®, Q7-4635®, Q7-4650®, Q7-4735®, Q7-4750®, Q7-4765®, MDX 4-4210"® et DC 3.6486® commercialisés par la Société DOW CORNING.

Le prépolymère de silicone utilisé est réticulable de préférence à des températures modérées telles que la température ambiante, en utilisant un catalyseur de réticulation biologiquement acceptable dans la matrice polymérique résultante et qui est compatible avec le composé actif dispersé dans cette dernière.

Par catalyseur de réticulation, on entend, selon la présente invention, l'association d'un agent de réticulation et d'un catalyseur.

Lorsque le prépolymère de silicone comporte des groupes hydroxy, tels que des groupes hydroxy terminaux, on peut citer, comme agent de réticulation, le tétrapropoxysilane [Si-(O-CH₂-CH₂-CH₃)₄] en association avec un catalyseur à base d'étain.

Lorsque le prépolymère de silicone comporte des groupes vinyliques, on peut réticuler ce dernier en présence d'un polymère de diméthyl-silicone en association avec un catalyseur tel qu'un catalyseur à base de platine.

Lorsque la matrice polymérique est à base d'un polyuréthanne, celle-ci est obtenue à partir d'un prépolymère du type polyester-polyol ou polyéther-polyol connu dans l'état de la technique. Parmi les polyesters-polyols on peut citer ceux obtenus par réaction d'alcools bi- ou trifonctionnels sur des acides tels que l'acide adipique, l'acide téréphtalique et de façon plus générale, tous autres acides plurifonctionnels. Parmi les polyéthers-polyols on peut citer ceux obtenus par alcoylation en faisant réagir des diols tels que l'éthylène glycol ou le propylène glycol ou des polyols tels que le triméthylolpropane, le glycérol, le pentaérythritol, le sorbitol etc. avec des oxydes tels que l'oxyde d'éthylène, l'oxyde de propylène ou leur mélange.

L'agent de polyaddition pour la formation des polyuréthannes est un isocyanate ou polyisocyanate, notamment le toluène diisocyanate, le diphénylméthane-4,4' diisocyanate, le naphtalène-1,5 diisocyanate ou l'isophorone diisocyanate.

Le catalyseur de réticulation ou l'agent de polyaddition est de préférence utilisé en une quantité telle que la réticulation ou la polyaddition ne soit pas complète, ceci de façon à ce que la couche de polymère présente en elle-même un caractère auto-adhésif satisfaisant pour ainsi avantageusement éviter que la couche support ne soit ultérieurement revêtue d'une couche adhésive.

Enfin, afin de renforcer la résistance à l'allongement de la matrice polymérique, la couche de polymère peut comporter une trame, constituée par exemple d'une feuille d'un matériau plastique perforée, d'une feuille d'un non-tissé perforée, ou d'un filet non étirable, le non-tissé ou le filet étant constitué de fibres naturelles ou synthétiques, telles que du polyamide, comme décrit dans le brevet français n° 92 05623 (FR-A-2 620 914).

La couche support ou couche occlusive des patchs selon la présente invention peut être constituée de tout matériau approprié imperméable aux composés actif contenu dans la couche de polymère adjacente.

. La couche support a non seulement pour fonction de supporter la couche de polymère mais également de servir de revêtement protecteur de celle-ci.

Elle peut être de même dimension que la couche de polymère ou de dimension plus grande de telle sorte qu'elle s'étende au-delà la périphérie de la couche de polymère et vers l'extérieur, de manière à ce que la surface qui entoure la couche de polymère puisse éventuellement recevoir des moyens adhésifs.

Parmi les matériaux appropriés pour la couche support, on peut citer des films de polyéthylène haute et basse densité, des films de polypropylène, de polychlorure de vinyle, de polyester tel que le polyphtalate d'éthylène, de copolymères éthylène-acétate de vinyle et de polyuréthanne. Ces matériaux peuvent également être présents sous forme stratifiée avec au moins une feuille de métal telle qu'une feuille d'aluminium. La couche support peut être de toute épaisseur appropriée qui procurera les fonctions de support et de protection souhaitées. De préférence, l'épaisseur de la couche support est comprise entre environ 0,2 mm et environ 1,5 mm.

Les patchs selon la présente invention peuvent, en outre, être protégés par la présence d'une couche détachable ou pelable de protection adjacente à la couche de polymère et/ou par un conditionnement dans un emballage approprié, notamment imperméable à l'eau et à la vapeur d'eau.

Lorsque la couche de polymère est protégée par une couche détachable de protection, celle-ci est enlevée au moment de l'utilisation. Elle peut être constituée en tout matériau imperméable au composé actif ainsi qu'à tout autre composant présent dans la matrice polymérique. Parmi les matériaux pouvant être utilisés, on peut citer de préférence une feuille de papier siliconée ou une feuille de matériau thermoplastique traitée pour la rendre anti-adhérente, par exemple à l'aide d'un vernis. De préférence, cette couche détachable de protection est constituée de polyéthylène.

De façon connue, les patchs selon la présente invention peuvent être découpés selon un contour approprié correspondant à la zone de surface de peau à traiter, par exemple sous forme de masque pour l'application sur le visage, notamment pour l'application sur les contours des yeux, sur les poches sous les yeux, sur le front. Bien entendu, les patchs selon la présente invention peuvent être découpés sous toute autre forme nécessaire pour une application sur une zone déterminée du corps. En général, la taille d'un patch conforme à l'invention est comprise entre 0,25 cm² et 500 cm². Ainsi, pour un patch destiné à la dépigmentation de taches pigmentées de la peau peut présenter une petite taille, inférieure à 1 cm², tandis qu'un patch à action amincissante peut présenter une grande surface, suffisante pour couvrir une partie d'une cuisse, par exemple.

Les patchs ainsi constitués et découpés peuvent être utilisés, après élimination de la couche détachable de protection, sur une surface de peau à traiter, en les appliquant directement sur une peau dont l'eau de transpiration permettra d'obtenir la libération désirée des composés actifs hydrophiles. Ils peuvent également être préalablement trempés dans de l'eau pendant un temps de préférence compris entre environ 5 et 30 secondes, ou être appliqués sur la peau après l'avoir préalablement mouillée par exemple à l'aide d'une éponge.

En général, l'humidité de transpiration de la peau est suffixante pour obtenir la libération du composé actif hydrophile sur la surface de la peau à traiter, à partir de la matrice polymérique hydrophobe le contenant.

Dans les patchs selon un mode de réalisation de la présente invention, la matrice polymérique constituant la couche de polymère est préparée par mélange intime sous agitation du prépolymère de silicone ou de polyuréthanne, des composés actifs hydrophiles, et de l'agent hydro-absorbant, tous deux sous forme de particules, ainsi que de l'huile comportant à l'état dissous les composés lipophiles mentionnés ci-dessus.

Au mélange ainsi obtenu, on ajoute alors à basse température, en général à température ambiante, soit un catalyseur de réticulation si le prépolymère est un polymère de silicone, soit un isocyanate ou polyisocyanate si le prépolymère est un polyester-polyol ou un polyéther-polyol.

Le mélange est alors introduit dans une trémie et versé sur une feuille de polyéthylène par exemple, constituant la pellicule de protection détachable ou pelable du patch. En aval de la trémie est disposé une racle permettant d'égaliser l'épaisseur de la couche de polymère de la matrice polymérique, cette épaisseur étant généralement comprise entre 0,1 mm et 12 mm.

On applique ensuite une feuille de trame tel que définie ci-dessus, puis avant calandrage, une feuille de la couche support ou occlusive qui peut être également une feuille de polyéthylène.

La polymérisation ou polyaddition est de préférence réalisée à température ambiante, ceci en vue de ne pas détériorer le ou les composés actifs. De façon générale, la polymérisation ou polyaddition est complète après environ 24 heures à température ambiante.

Après calandrage et avant que la polymérisation ou polyaddition ne soit complète, la structure composite obtenue peut être immédiatement découpée aux formes voulues, ce qui permet d'obtenir des bords pincés évitant tout phénomène de coulage.

Les exemples suivants permettent d'illustrer la présente invention.

### EXEMPLE 1

A 8 g de polyacrylate en poudre (*Aquakeep®* commercialisé par la Société NORSOLOR), on ajoute 10 g d'huile d'amande douce contant 0,2 g de all trans-rétinol et 2 g de vitamine C microcristalline. On ajoute 43 g d'organopolysiloxane *DC3.6486®* (commercialisé par la Société DOW CORNING). Sous agitation à 1.500 tours/min., on ajoute 1,7 g de catalyseur de réticulation *Medical Grade Curing Agent* et on maintient l'agitation pendant quelques minutes.

Le produit ainsi homogénéisé est introduit dans une trémie et est étalé à l'aide d'une racle en une couche de 0,8 mm d'épaisseur sur une feuille de polyéthylène d'une épaisseur de 200 µm. Cette feuille peut être préalablement traitée en surface pour réduire son adhérence. Sur la feuille de polyéthylène ainsi revêtue, on applique une trame constituée par un filet de polyamide ou de polyéthylène comportant des mailles d'une ouverture de 1 mm et d'une épaisseur de 0,3 mm.

On applique ensuite un film de polyéthylène (sans traitement anti-adhérent) de 30 µm d'épaisseur qui constitue la couche support ou occlusive du patch, et on procède au calandrage de l'ensemble. On obtient ainsi un ensemble comportant une couche support occlusive et une couche de polymère auto-adhésive formée d'une matrice en polymère de silicone partiellement réticulé, cet ensemble comprenant en outre, une couche détachable de protection.

A partir de cet ensemble, on peut réaliser par découpe, différentes formes de patch en fonction des utilisations souhaitées.

Les patchs après découpe sont ensuite conditionnés dans des sachets de polyéthylène. Lors de l'utilisation, le patch, après élimination de la couche détachable de protection, est appliqué directement sur le contour d'un oeil, par exemple pendant une période de 30 minutes environ. Après avoir retiré le patch, on constate visuellement que le contour de l'oeil traité à l'aide de ce patch, présente de manière sensible un aspect plus lisse et plus reposé que celui de l'autre oeil non traité. Ce patch présente un bon pouvoir adhésif sur la peau

### EXEMPLE 2

A 8 g de polyacrylate en poudre (*Aquakeep®* commercialisé par la Société NORSOLOR), on ajoute 12 g d'huile de ricin contant 0,3 g de D-α-tocophérol et 2 g de lactate de sodium microcristallin. On ajoute 43 g d'organopolysiloxane *DC3.6486*® commercialisé par la Société Dow CORNING). Sous agitation à 1.500 tours/min., on ajoute 1,7g de catalyseur de réticulation *Medical Grade Curing Agent* et on maintient l'agitation pendant quelques minutes.

On confectionne un patch de manière analogue à celui de l'exemple 1. Après l'application de ce patch sur la peau pendant 12 heures, on constate encore un bon pouvoir adhésif.

Un test de conservation à 45°C pendant 48 heures démontre que ni l'huile de ricin ni le tocophérol ne s'est dégradé.

### EXEMPLE 3

A 8 g de polyacrylate en poudre (*Aquakeep®* commercialisé par la Société NORSOLOR), on ajoute 10 g d'huile de jojoba, 0,6 g de β-carotène et 1,5 g d'hydroxyproline microcristallin. On ajoute 43 g d'organopolysiloxane *DC3.6486*® (commercialisé par la Société Dow CORNING). Sous agitation à 1.500 tours/min., on ajoute 1,7 g de son catalyseur de réticulation *Medical Grade Curing Agent* et on maintient l'agitation pendant quelques minutes.

De manière analogue à l'exemple 1, on confectionne un patch, dont l'action est réparatrice de la peau photoendommagée, émolliente et cicatrisante. Le pouvoir adhésif de ce patchsur la peau, évalué au bout de 12 heures, s'est avéré satisfausant.

### EXEMPLE 4

A 10 g d'huile de sésame contenant 0,2 g de nicotinate d'hexyle, on ajoute 1,5 g d'urée et 0,5 g de caféine en poudre. On ajoute 43 g d'organopolysiloxane *DC3.6486®* (commercialisé par la Société Dow CORNING). Sous agitation à 1.500 tours/min., on ajoute 1,7 g de son catalyseur de réticulation *Medical Grade Curing Agent* et on maintient l'agitation pendant quelques minutes.

On confectionne un patch de manière analogue à l'exemple 1. Après application pendant 15 minutes, ce patch augmente la microcirculation vasculaire de la peau et favorise le métabolisme des graisses sous-cutanées.

## Revendications

1. Patch, comportant une couche de polymère hydrophobe, fixée sur une couche support et contenant :
a) des premières particules d'au moins un composé actif hydrosoluble,
b) des deuxièmes particules d'huile,
c) au moins un composé actif liposoluble,
**caractérisé en ce que** la couche de polymère contient, en outre, des troisièmes particules d'un agent hydro-absorbant, dispersées de façon homogène dans la couche de polymère.

2. Patch selon la revendication 1, **caractérisé en ce que** le composé actif liposoluble est solubilisé dans l'huile.

3. Patch selon la revendication 1, **caractérisé en ce que** le composé actif liposoluble est sous forme particulaire, dispersé dans la couche de polymère.

4. Patch selon l'une quelconque des revendications 1 à 3, **caractérisé en ce que** l'agent hydro-absorbant est choisi parmi les polyacrylates réticulés superabsorbants, l'alcool polyvinylique, les polymères carboxyvinyliques, les dérivés semi-synthétiques de là cellulose, les amidons, les gommes de guar, arabique ou adragante, la caséine, les phytocolloïdes, les fibres de coton et la gélatine..

5. Patch selon l'une quelconque des revendications 1 à 4, **caractérisé en ce que** l'agent hydro-absorbant est sous forme de particules d'une poudre d'émulsion lyophilisée ou atomisée.

6. Patch selon l'une quelconque des revendications précédentes, **caractérisé en ce que** l'agent hydro-absorbant est présent dans la couche de polymère en une proportion allant d'environ 0,1 % à environ 30 % en poids par rapport au poids total de ladite couche.

7. Patch selon l'une quelconque des revendications précédentes, **caractérisé en ce que** les particules de l'agent hydro-absorbant ont une taille moyenne comprise entre environ 0,2 µm et environ 1,5 mm.

8. Patch selon l'une quelconque des revendications précédentes, **caractérisé en ce que** l'huile est choisie parmi les huiles minérales, animales, végétales ou synthétiques ou leurs mélanges.

9. Patch selon l'une quelconque des revendications précédentes, **caractérisé en ce que** l'huile est présente dans la couche de polymère dans une proportion allant de 0,1 % à 30 %, par rapport au poids total de la couche de polymère.

10. Patch selon l'une des revendications précédentes, **caractérisé en ce que** le composé actif liposoluble est présent dans la couche de polymère en une proportion allant de 0,01 % à 20 % en poids, par rapport au poids total de l'huile.

11. Patch selon l'une quelconque des revendications précédentes, **caractérisé en ce que** le composé actif liposoluble, est choisi parmi les composés suivants : D-α-tocophérol, DL-α-tocophérol, acétate de D-α-tocophérol, acétate de DL-α-tocophérol, palmitate d'ascorbyle, vitamine F et glycérides de vitamine F, vitamine D, vitamine D₂, vitamine D₃, rétinol, esters de rétinol, palmitate de rétinol, propionate de rétinol, β-carotène, D-panthénol, farnesol, acétate de farnesyle ; huiles de jojoba et de cassis riches acides gras essentiels ; acide salicylique et ses esters, acide n-octanoyl-5 salicylique et ses esters, alkylesters d'α-hydroxyacides tels que l'acide citrique, acide lactique, acide glycolique ; acide asiatique, acide madécassique, asiaticoside, extrait total de centella asiatica, acide β-glycyrrhétinique, α-bisabolol, céramides comme le 2 oleoylamino-1,3 octadécane ; phytanetriol, phospholipides d'origine marine riches en acides gras essentiels polyinsaturés, éthoxyquine ; extrait de romarin, extrait de mélisse, quercetine, extrait de microalgues séchées, anti-inflammatoires stéroïdiens.

12. Patch selon l'une quelconque des revendications précédentes, **caractérisé en ce que** le composé actif hydrosoluble est présent dans la couche de polymère en une proportion allant de 0,2 % à 20 % en poids par rapport au poids total de ladite couche.

13. Patch selon l'une quelconque des revendications précédentes, **caractérisé en ce que** le composé actif hydrosoluble est sous forme particulaire dont la taille moyenne des particules est comprise entre 0,2 µm et 0,5 mm.

14. Patch selon l'une quelconque des revendications précédentes, **caractérisé en ce que** le composé actif hydrosoluble est choisi parmi les composés suivants : l'acide ascorbique et ses sels biologiquement compatibles, les enzymes, des antibiotiques, les composants à effet tenseur, les α-hydroxy acides et leurs sels, les polyacides hydroxylés, les sucroses et leurs dérivés, l'urée, les aminoacides, les oligopeptides, les extraits végétaux hydrosolubles, et de levures, les hydrolysats de protéines, l'acide hyaluronique, les mucopolysaccharides, les vitamines B₂, B₆, H, PP, le panthénol, l'acide folique, l'acide acétyl salicylique, l'allantoïne, l'acide glycyrrhétique, l'acide kojique, l'hydroquinone.

15. Patch selon l'une quelconque des revendications précédentes, **caractérisé en ce que** la couche de polymère hydrophobe est à base d'un polymère de silicone ou d'un polyuréthanne.

16. Patch selon la revendication 15, **caractérisé en ce que** la couche polymérique à base de polymère de silicone est obtenue par réticulation d'un organopolysiloxane linéaire substitué sur l'atome de silicium par des groupements choisis parmi un groupement alkyle en C₁-C₆, aryle ou ar(alkyle en C₁-C₂), les atomes de silicium terminaux étant trisubstitués par un groupement alkyle en C₁-C₆.

17. Patch selon la revendication 16, **caractérisé en ce que** le polymère de silicone comporte des motifs : 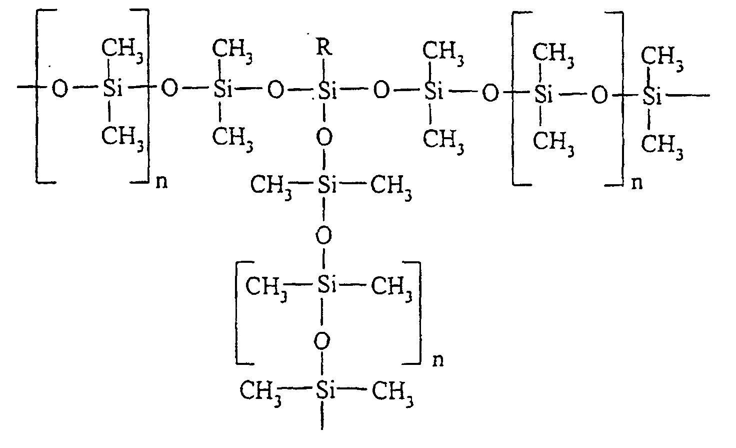 dans lesquels :
R représente un groupe alkyle ou alkoxy contenant de 1 à 7 atomes de carbone, un groupe vinyle ou phényle, et dans lesquels n est choisi dans la gamme allant d'environ 100 à environ 5.000.

18. Patch selon la revendication 15, **caractérisé en ce que** la couche polymérique à base d'un polyuréthanne est obtenue par polyaddition d'un polyester-polyol ou d'un polyéther-polyol en présence d'un isocyanate ou polyisocyanate choisi parmi le toluène diisocyanate, le diphényl-4,4' diisocyanate, le naphtalène-1,5 diisocyanate et l'isophorone diisocyanate.

19. Patch selon l'une quelconque des revendications précédentes, **caractérisé en ce qu'**il renferme, en outre, une trame intercalée entre la couche de polymère et la couche support.

20. Patch selon la revendication 19, **caractérisé en ce que** la trame est choisie parmi une feuille d'un matériau plastique perforée, une feuille d'un non-tissé de fibres naturelles ou synthétiques et un filet non étirable en fibres naturelles ou synthétiques.

21. Patch selon l'une quelconque des revendications précédentes, **caractérisé en ce que** l'épaisseur de la couche de polymère est comprise entre environ 0,1 mm et 12 mm.

22. Patch selon l'une quelconque des revendications précédentes, **caractérisé en ce que** la couche support est constituée d'un polymère choisi parmi les polyéthylènes haute et basse densité, les polypropylènes, les polychlorure de vinyle, les copolymères d'éthylène et d'acétate de vinyle, les polyesters et les polyuréthannes.

23. Patch selon l'une quelconque des revendications précédentes, **caractérisé en ce que** l'épaisseur de la couche support est comprise entre environ 0,2 mm et 1,5 mm.

24. Patch selon l'une quelconque des revendications précédentes, **caractérisé par le fait qu'**il est auto-adhésif.

## Patentansprüche

1. Pflaster, das eine hydrophobe Polymerschicht aufweist, die an einer Trägerschicht fixiert ist und enthält:
a) erstens Partikel mindestens eines wasserlöslichen Wirkstoffes,
b) zweites Ölpartikel,
c) mindestens einen fettlöslichen Wirkstoff,
**dadurch gekennzeichnet, dass** die Polymerschicht ferner drittens Partikel eines wasserabsorbierenden Stoffs enthält, die homogen in der Polymerschicht dispergiert sind.

2. Pflaster nach Anspruch 1, **dadurch gekennzeichnet, dass** der fettlösliche Wirkstoff in dem Öl solubilisiert ist.

3. Pflaster nach Anspruch 1, **dadurch gekennzeichnet, dass** der fettlösliche Wirkstoff in Partikelform vorliegt und in der Polymerschicht dispergiert ist.

4. Pflaster nach einem der Ansprüche 1 bis 3, **dadurch gekennzeichnet, dass** der wasserabsorbierende Stoff unter den superabsorbierenden vernetzten Polyacrylaten, Polyvinylalkohol, Carboxyvinylpolymeren, halbsynthetischen Cellulosederivaten, Stärkeverbindungen, Guargummi, Gummi arabicum oder Tragant, Casein, Phytokolloiden, Baumwollfasern und Gelatine ausgewählt ist.

5. Pflaster nach einem der Ansprüche 1 bis 4, **dadurch gekennzeichnet, dass** der wasserabsorbierende Stoff in Form von Partikeln eines Pulvers einer gefriergetrockneten oder zerstäubten Emulsion vorliegt.

6. Pflaster nach einem der vorhergehenden Ansprüche, **dadurch gekennzeichnet, dass** der wasserabsorbierende Stoff in der Polymerschicht in einem Mengenanteil von etwa 0,1 bis etwa 30 Gew.-%, bezogen auf das Gesamtgewicht der Schicht, enthalten ist.

7. Pflaster nach einem der vorhergehenden Ansprüche, **dadurch gekennzeichnet, dass** die Partikel des wasserabsorbierenden Stoffs eine mittlere Größe von etwa 0,2 µm bis 1,5 mm besitzen.

8. Pflaster nach einem der vorhergehenden Ansprüche, **dadurch gekennzeichnet, dass** das Öl unter den Mineralölen, tierischen Ölen, pflanzlichen Ölen oder synthetischen Ölen oder deren Gemischen ausgewählt ist.

9. Pflaster nach einem der vorhergehenden Ansprüche, **dadurch gekennzeichnet, dass** das Öl in der Polymerschicht in einer Menge von 0,1 bis 30 %, bezogen auf das Gesamtgewicht der Polymerschicht, enthalten ist.

10. Pflaster nach einem der vorhergehenden Ansprüche, **dadurch gekennzeichnet, dass** der fettlösliche Wirkstoff in der Polymerschicht in einem Mengenanteil von 0,01 bis 20 Gew.-%, bezogen auf das Gesamtgewicht des Öls, enthalten ist.

11. Pflaster nach einem der vorhergehenden Ansprüche, **dadurch gekennzeichnet, dass** der fettlösliche Wirkstoff unter den folgenden Verbindungen ausgewählt ist: D-α-Tocopherol, DL-α-Tocopherol, D-α-Tocopherolacetat, DL-α-Tocopherolacetat, Ascorbylpalmitat, Vitamin F und Glyceriden von Vitamin F, Vitamin D, Vitamin D₂, Vitamin D₃, Retinol, Retinolestern, Retinolpalmitat, Retinolpropionat, β-Carotin, D-Panthenol, Farnesol, Farnesylacetat; Jojobaöl und Johannisbeerkernöl, die einen hohen Gehalt an essentiellen Fettsäuren aufweisen; Salicylsäure und ihren Estern, 5-n-Octanoylsalicylsäure und ihren Estern, Alkylestern von α-Hydroxysäuren, wie Citronensäure, Milchsäure, Glykolsäure; Asiatsäure, Madecassäure, Asiaticosid, dem Gesamtextrakt von Centella Asiatica, β-Glycyrrhetinsäure, α-Bisabolol, Ceramiden, wie 2-Oleylamino-1,3-octadecan; Phytantriol, Phospholipiden mariner Herkunft, die einen hohen Gehalt an mehrfach ungesättigten essentiellen Fettsäuren aufweisen, Ethoxyquin; Rosmarinextrakt, Melissenextrakt, Quercetin, Extrakten aus getrockneten Mikroalgen und steroidalen entzündungshemmenden Wirkstoffen.

12. Pflaster nach einem der vorhergehenden Ansprüche, **dadurch gekennzeichnet, dass** der wasserlösliche Wirkstoff in der Polymerschicht in einem Mengenanteil von 0,2 bis 20 Gew.-%, bezogen auf das Gesamtgewicht der Schicht, enthalten ist.

13. Pflaster nach einem der vorhergehenden Ansprüche, **dadurch gekennzeichnet, dass** der wasserlösliche Wirkstoff in Partikelform vorliegt, wobei die mittlere Größe der Partikel im Bereich von 0,2 µm bis 0,5 mm liegt.

14. Pflaster nach einem der vorhergehenden Ansprüche, **dadurch gekennzeichnet, dass** der wasserlösliche Wirkstoff unter den folgenden Verbindungen ausgewählt ist: Ascorbinsäure und ihren biologisch verträglichen Salzen, Enzymen, Antibiotika, Verbindungen mit straffender Wirkung, α-Hydroxysäuren und ihren Salzen, hydroxylierten Polysäuren, Disacchariden und ihren Derivaten, Harnstoff, Aminosäuren, Oligopeptiden, wasserlöslichen pflanzlichen Extrakten und Hefeextrakten, Proteinhydrolysaten, Hyaluronsäure, Mucopolysacchariden, Vitaminen B₂, B₆, H, PP, Panthenol, Folsäure, Acetylsalicylsäure, Allantoin, Glycyrrhetinsäure, Kojisäure und Hydrochinon.

15. Pflaster nach einem der vorhergehenden Ansprüche, **dadurch gekennzeichnet, dass** die hydrophobe Polymerschicht auf einem Siliconpolymer oder einem Polyurethanpolymer basiert.

16. Pflaster nach Anspruch 15, **dadurch gekennzeichnet, dass** die Polymerschicht auf der Basis eines Siliconpolymers durch Vernetzung eines linearen Organopolysiloxans hergestellt wird, das am Siliciumatom mit Gruppen substituiert ist, die unter den C₁₋₆-Alkylgruppen, Arylgruppen oder Ar(C₁₋₂-alkyl)gruppen ausgewählt sind, wobei die endständigen Siliciumatome mit einer C₁₋₆-Alkylgruppe dreifach substituiert sind.

17. Pflaster nach Anspruch 16, **dadurch gekennzeichnet; dass** das Siliconpolymer die folgenden Einheiten enthält: 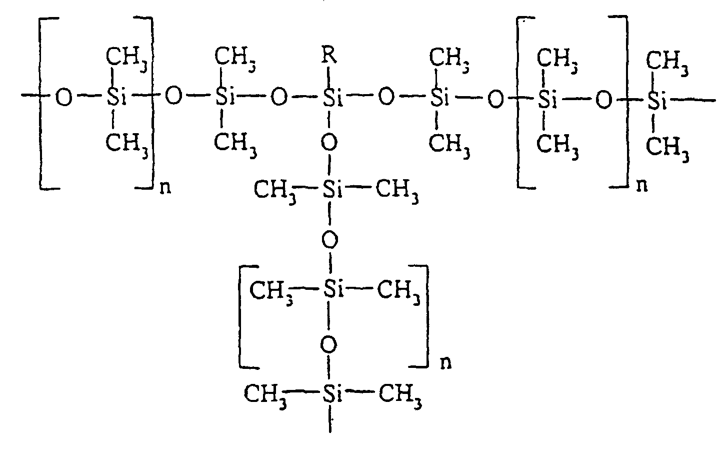 worin bedeuten:
R eine Alkylgruppe oder Alkoxygruppe mit 1 bis 7 Kohlenstoffatomen, eine Vinylgruppe oder eine Phenylgruppe, wobei n im Bereich von etwa 100 bis etwa 5 000 ausgewählt ist.

18. Pflaster nach Anspruch 15, **dadurch gekennzeichnet, dass** die Polymerschicht auf der Basis eines Polyurethan durch Polyaddition eines Polyesterpolyols oder Polyetherpolyols in Gegenwart eines Isocyanats oder Polyisocyanats hergestellt wird, das unter Toluoldiisocyanat, Diphenyl-4,4'-diisocyanat, 1,5-Naphthalindiisocyanat und Isophorondiisocyanat ausgewählt ist.

19. Pflaster nach einem der vorhergehenden Ansprüche, **dadurch gekennzeichnet, dass** es ferner zwischen der Polymerschicht und der Trägerschicht eine dazwischenliegende Stützschicht aufweist.

20. Pflaster nach Anspruch 19, **dadurch gekennzeichnet, dass** die Stützschicht unter einer Folie aus einem perforierten Kunststoff, aus einer Folie aus einem Vliesstoff aus natürlichen oder synthetischen Fasern und einem nicht dehnbaren Netz aus natürlichen oder synthetischen Fasern ausgewählt ist.

21. Pflaster nach einem der vorhergehenden Ansprüche, **dadurch gekennzeichnet, dass** die Dicke der Polymerschicht im Bereich von etwa 0,1 bis 12 mm gewählt ist.

22. Pflaster nach einem der vorhergehenden Ansprüche, **dadurch gekennzeichnet, dass** die Trägerschicht aus einem Polymer besteht, das unter den Polyethylenen hoher und niedriger Dichte, Polypropylenen, Polyvinylchlorid, Copolymeren von Ethylen und Vinylacetat, Polyestern und Polyurethanen ausgewählt ist.

23. Pflaster nach einem der vorhergehenden Ansprüche, **dadurch gekennzeichnet, dass** die Dicke der Trägerschicht im Bereich von etwa 0,2 bis 1,5 mm liegt.

24. Pflaster nach einem der vorhergehenden Ansprüche, **dadurch gekennzeichnet, dass** es selbsthaftend ist.

## Claims

1. Patch, comprising a hydrophobic polymer layer, bound to a support layer and containing:
a) first particles of at least one water-soluble active compound,
b) second particles of oil,
c) at least one liposoluble active compound,
**characterized in that** the polymer layer also contains third particles of a water-absorbing agent, dispersed homogeneously in the polymer layer.

2. Patch according to Claim 1, **characterized in that** the liposoluble active compound is dissolved in the oil.

3. Patch according to Claim 1, **characterized in that** the liposoluble active compound is in particulate form, dispersed in the polymer layer.

4. Patch according to any one of Claims 1 to 3, **characterized in that** the water-absorbing agent is chosen from superabsorbent crosslinked polyacrylates, polyvinyl alcohol, carboxyvinyl polymers, semisynthetic cellulose derivatives, starches, guar gum, gum arabic or gum tragacanth, casein, phytocolloids, cotton fibres and gelatin.

5. Patch according to any one of Claims 1 to 4, **characterized in that** the water-absorbing agent is in the form of particles of a powder of freeze-dried or sprayed emulsion.

6. Patch according to any one of the preceding claims, **characterized in that** the water-absorbing agent is present in the polymer layer in a proportion ranging from about 0.1% to about 30% by weight relative to the total weight of the said layer.

7. Patch according to any one of the preceding claims, **characterized in that** the particles of the water-absorbing agent have an average size of between about 0.2 µm and about 1.5 mm.

8. Patch according to any one of the preceding claims, **characterized in that** the oil is chosen from mineral, animal, plant or synthetic oils or mixtures thereof.

9. Patch according to any one of the preceding claims, **characterized in that** the oil is present in the polymer layer in a proportion ranging from 0.1% to 30% relative to the total weight of the polymer layer.

10. Patch according to one of the preceding claims, **characterized in that** the liposoluble active compound is present in the polymer layer in a proportion ranging from 0.01% to 20% by weight relative to the total weight of the oil.

11. Patch according to any one of the preceding claims, **characterized in that** the liposoluble active compound is chosen from the following compounds: D-α-tocopherol, DL-α-tocopherol, D-α-tocopheryl acetate, DL-α-tocopheryl acetate, ascorbyl palmitate, vitamin F and vitamin F glycerides, vitamin D, vitamin D₂, vitamin D₃, retinol, retinol esters, retinyl palmitate, retinyl propionate, β-carotene, D-panthenol, farnesol, farnesyl acetate; jojoba oils and blackcurrant oils rich in essential fatty acids; salicylic acid and esters thereof, 5-n-octanoylsalicylic acid and esters thereof, alkyl esters of α-hydroxy acids such as citric acid, lactic acid, glycolic acid; asiatic acid, madecassic acid, asiaticoside, total extract of Centella asiatica, β-glycyrrhetinic acid, α-bisabolol, ceramides such as 2-oleoylamino-1,3-octadecane; phytanetriol, phospholipids of marine origin which are rich in polyunsaturated essential fatty acids, ethoxyquine; extract of rosemary, extract of balm, quercetin, extract of dried microalgae, steroidal anti-inflammatory agents.

12. Patch according to any one of the preceding claims, **characterized in that** the water-soluble active compound is present in the polymer layer in a proportion ranging from 0.2% to 20% by weight relative to the total weight of the said layer.

13. Patch according to any one of the preceding claims, **characterized in that** the water-soluble active compound is in a particulate form in which the average particle size is between 0.2 µm and 0.5 mm.

14. Patch according to any one of the preceding claims, **characterized in that** the water-soluble active compound is chosen from the following compounds: ascorbic acid and biologically compatible salts thereof, enzymes, antibiotics, components with a surfactant effect, α-hydroxy acids and salts thereof, hydroxylated polyacids, sucroses and derivatives thereof, urea, amino acids, oligopeptides, water-soluble plant extracts and yeasts, protein hydrolysates, hyaluronic acid, mucopolysaccharides, vitamins B₂, B₆, H and PP, panthenol, folic acid, acetylsalicylic acid, allantoin, glycyrrhetinic acid, kojic acid, hydroquinone.

15. Patch according to any one of the preceding claims, **characterized in that** the hydrophobic polymer layer is based on a silicone polymer or a polyurethane.

16. Patch according to Claim 15, **characterized in that** the polymer layer based on silicone polymer is obtained by crosslinking a linear organopolysiloxane substituted on the silicon atom with groups chosen from a C₁-C₆ alkyl, aryl or ar(C₁-C₂ alkyl) group, the end silicon atoms being trisubstituted with a C₁-C₆ alkyl group.

17. Patch according to Claim 16, **characterized in that** the silicone polymer contains units: 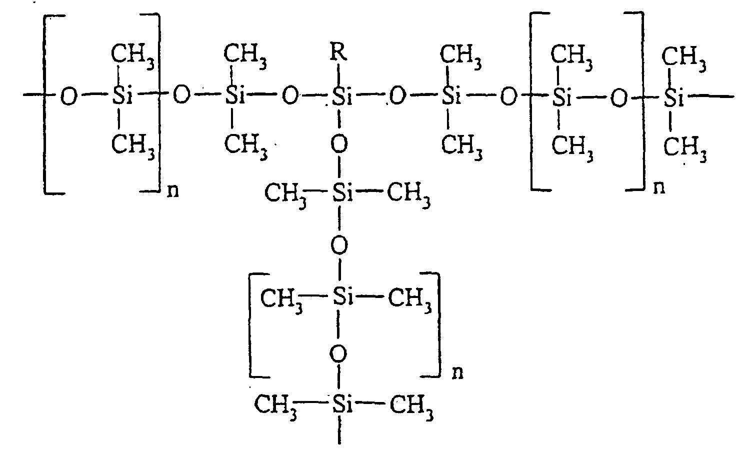 in which:
R represents an alkyl or alkoxy group containing from 1 to 7 carbon atoms, a vinyl or phenyl group, and in which n is chosen in the range from about 100 to about 5000.

18. Patch according to Claim 15, **characterized in that** the polymer layer based on a polyurethane is obtained by polyaddition of a polyester-polyol or of a polyether-polyol in the presence of an isocyanate or polyisocyanate chosen from toluene diisocyanate, diphenyl 4,4'-diisocyanate, naphthalene 1,5-diisocyanate and isophorone diisocyanate.

19. Patch according to any one of the preceding claims, **characterized in that** it also contains a frame inserted between the polymer layer and the support layer.

20. Patch according to Claim 19, **characterized in that** the frame is chosen from a perforated sheet of a plastic material, a sheet of a nonwoven material of natural or synthetic fibres and a non-stretchable gauze made of natural or synthetic fibres.

21. Patch according to any one of the preceding claims, **characterized in that** the thickness of the polymer layer is between about 0.1 mm and 12 mm.

22. Patch according to any one of the preceding claims, **characterized in that** the support layer consists of a polymer chosen from high- and low-density polyethylenes, polypropylenes, polyvinyl chlorides, copolymers of ethylene and of vinyl acetate, polyesters and polyurethanes.

23. Patch according to any one of the preceding claims, **characterized in that** the thickness of the support layer is between about 0.2 mm and 1.5 mm.

24. Patch according to any one of the preceding claims, **characterized in that** it is self-adhesive.
